# EUROPÄISCHE PATENTSCHRIFT

(11) **EP 1 179 991 B1**
(45) Veröffentlichungstag und Bekanntmachung des Hinweises auf die Patenterteilung: **10.05.2006**
(21) Anmeldenummer: 00916695.0
(22) Anmeldetag: 27.03.2000
(51) Int. Cl.: A61B 5/0225

(54) **KONTINUIERLICHES NICHT-INVASIVES BLUTDRUCKMESSGERÄT**
CONTINUOUS NON-INVASIVE SPHYGMOMANOMETER
TENSIOMETRE NON INVASIF A MESURE CONTINUE

(30) Priorität: 30.03.1999 AT 57699
(43) Veröffentlichungstag der Anmeldung: 20.02.2002
(60) Teilanmeldung: 05000042.1
(73) Patentinhaber: CNSystems Medizintechnik GmbH, 8020 Graz (AT)
(72) Erfinder: FORTIN, Jürgen, 8020 Graz (AT); SKRABAL, Falko, 8043 Graz (AT)
(74) Vertreter: Babeluk, Michael
(86) Internationale Anmeldenummer: PCT/AT2000/000073
(87) Internationale Veröffentlichungsnummer: WO 2000/059369

(56) Entgegenhaltungen:
- EP-A- 0 395 519
- EP-A- 0 426 572
- WO-A-94/22364
- WO-A-95/00070
- AT-B- 391 262
- DE-A- 3 829 456
- US-A- 4 406 289
- US-A- 4 771 790
- US-A- 4 850 369
- US-A- 5 651 370
- US-A- 5 662 092

## Beschreibung

Die Erfindung betrifft ein Blutdruckmessgerät für die kontinuierliche, plethysmographische Blutdruckmessung, mit zumindest einem aufblasbaren Druckpolster, der an einem eine Arterie enthaltenden Körperteil befestigbar ist, mit dem Körperteil zugeordneten Arteriensignalaufnehmern zum Bestimmen des Durchflusses, mit einer ventilgesteuerten Druckkammer, welche mit einer Gasquelle und mit dem aufblasbaren Druckpolster in Verbindung steht, mit einem Druckaufnehmer zur Messung des Druckes in der Druckkammer bzw. dem Druckpolster und mit einem Rechner, wobei die Ventilsteuerung der Druckkammer in Abhängigkeit des Signals der Arteriensignalaufnehmer erfolgt.

In der Medizin besteht die Notwendigkeit der häufigen, wenn möglich auch kontinuierlichen Blutdruckmessung. Dafür wurden in jüngster Zeit neue Geräte entworfen. Eine wesentliche Neuerung brachte die Methode von Penaz (Digest of the 10^{th} International Conference on Medical and Biological Engineering 1973 Dresden), bei der ein Finger durchleuchtet wird und durch eine Servoregelung der registrierte Fluss konstant gehalten wird.

Diese photoplethysmographische Methode wurde von einigen anderen aufgegriffen (Yamakoshi, Wesseling, TNO). Die EP 0 537 383 (TNO) vom 21. April 1993 zeigt eine aufblasbare Fingermanschette für die nicht-invasive kontinuierliche Blutdrucküberprüfung. Der aufblasbare zylindrische Raum ist pneumatisch mit einer Fluidquelle verbunden. Eine Infrarotlichtquelle und ein Detektor sind beidseitig des Fingers innerhalb des festen Zylinders positioniert. Es ist ein Ventil zum Füllen des Zylinders mit Gas vorgesehen. Es sind elektrische Kabel für die Infrarotlichtquelle und des Detektors hindurchgeführt. Die US 4,510,940 A (WESSELING) 16. April 1985 und die US 4,539,997 A (WESSELING) 10. September 1985 zeigen Vorrichtungen zur kontinuierlichen nicht-invasiven Messung des Blutdruckes. Es sind eine fluidgefüllte Manschette, eine Lichtquelle, ein Lichtdetektor und ein Differenzdruckverstärker vorgesehen. Die US 4,406,289 A (WESSELING) 27. September 1983 zeigt ebenfalls eine solche Vorrichtung gemäß dem Stand der Technik.

Ein Hauptproblem der bekannten Verfahren und Vorrichtungen liegt einerseits in den verwendeten Manschetten, die sehr genau platziert werden müssen, sehr störanfällig und wenig haltbar sind, andererseits bei dem verwendeten, sehr teuer herzustellenden Proportionalventil (US 4,406,289 A) und weiters in der Eichung des Gerätes, das zwar sehr genau die relativen Schwankungen des Blutdruckes anzeigen kann, bei der Absolutmessung jedoch sehr von den tatsächlichen intraarteriellen Werten abweicht. Üblicherweise wird in den bisher verwendeten Proportionalventilen entweder a) eine Kippstange (Flapper) verwendet, die durch einen Elektromagneten abwechselnd in die eine oder andere Richtung bewegt werden kann, oder b) ein elektromagnetischer Shaker verwendet. Bei beiden diesen Proportionalventilen ist ein ständiger Gasfluss durch die Druckkammer gegeben, da immer ein Teil des Ventils geöffnet ist. Entweder ist die Auslassöffnung ins Freie, bzw. Einlassöffnung von der Gaszufuhr freigegeben. Es gibt keine Stellung des Ventils, bei dem beide, Einlass- und Auslassöffnung gleichzeitig geschlossen sind.

Dadurch ergibt sich ein sehr hoher Gasverbrauch, der zwar bei stationären Geräten wenig relevant ist, aber bei ambulant zu tragenden Geräten deutlich zu tragen kommt. Ein weiterer Nachteil ist die Verwendung von Druckerzeugungssystemen (in der Regel Pumpen und Kompressoren), die einen Druckfluss ohne Welligkeit erzeugen müssen, da diese Welligkeit das Messsignal beeinflussen würde. Pumpen bzw. Kompressoren die einen konstanten und glatten Luftfluss erzeugen, sind in der Regel teuer und verbrauchen mehr Energie als Druckerzeugungssysteme, die einen Druck liefern, der eine bestimmte Schwelle nicht unterschreiten darf. Das Gewicht bzw. der Energieverbrauch des Gerätes ist deutlich erhöht.

Ein weiterer Nachteil der verwendeten Methoden liegt darin, dass diese bisher verwendeten Methoden ausschließlich am Finger verwendet werden, die Fingerarterien jedoch schon zu den kleinen Arterien zählen, die im Fluss vom Körper z.B. von der Temperatur der Finger geregelt werden, so dass der Druck in diesen Arterien nicht mehr dem Druck in den großen Arterien entspricht, für die sich der Mediziner in erster Linie interessiert. Aus diesem Grund geben die bisher verwendeten Geräte (z.B. der Finapres der Fa. OHMEDA) zwar sehr gut die Relativschwankungen des Blutdruckes an, jedoch nicht Absolutwerte des Druckes, so dass das Gerät Finapres auch vom Markt genommen wurde.

Ein weiteres bestehendes Blutdruckmessgerät verwendet im Wesentlichen die Planartonometrie. Dabei wird ein Array von sehr kleinen Druckaufnehmern, die in Silikon gebettet sind, über einen Luftdruckbalg auf die Arterie gebracht, wobei ein Computer unter den Drucksensoren den heraussucht, der das beste Signal liefert. Der Druck im Balg wird nach Erreichen eines guten Signals nicht mehr geändert, die Eichung der Druckkurve erfolgt über einmalige oder mehrfache Messung des oszillometrischen Blutdruckes, der am selben Oberarm intermittierend gemessen werden kann. Das Aufbringen eines harten Gegenstandes, nämlich des Arrays auf die Arterie verformt dieselbe in nicht kontrollierbarer Weise, so dass die Druckwerte, die dieses Gerät ausgibt, sehr stark von den intraarteriellen Werten abweichen. (Zorn et al., Blood Pressure Monitoring 2:185, 1997) Die genaue Analyse der Druckkurven kann zusätzlich auch mit Hilfe eines erweiterten Windkesselmodells in bekannter Weise verwendet werden, um die Compliance der großen und kleinen Gefäße zu errechnen, wie das von Watt und Burrus gezeigt wurde. Weiters kann z.B. mit Frequenzanalysen auch der Druck in der zentralen Aorta rechnerisch ermittelt werden oder auch ein sogenannter Augmentationsindex errechnet werden, der sehr gut die tatsächliche mechanische Belastung des Herzens und Gefäßsysteme widerspiegelt. Bisher wurde dazu die sogenannte Aplanationstonometrie verwendet, bei der ein harter Druckaufnehmer händisch oder per Mikrometerschraube auf die Arterie gebracht wurde, um so die Arterienwand zu entlasten. Das hat bisher den Nachteil gebracht, dass der Druck mit dem Druckaufnehmer auf der Arterie lag, nicht bekannt war, und dass es äußerst mühsam war, händisch die Arterie genau zu treffen.

Die gegenständliche Erfindung bezweckt die Vermeidung der genannten Schwierigkeiten durch Schaffung eines neuen Blutdruckmessgerätes.

Erfindungsgemäß wird das dadurch erreicht, dass die Druckkammer mit je einem separaten Einlass- und Auslassventil ausgestattet ist.

Das Blutdruckmessgerät gemäß der Erfindung wird anhand der Abbildungen näher beschrieben.

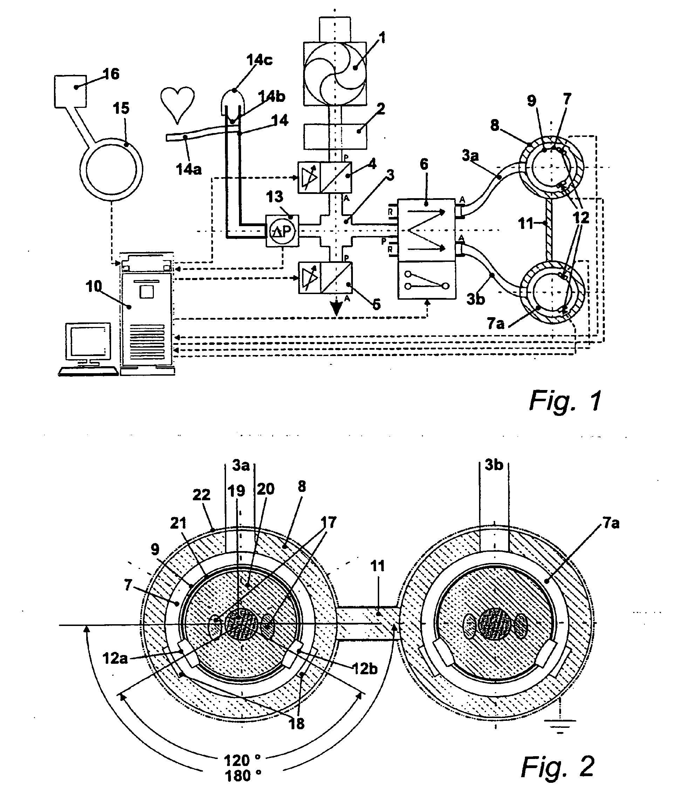

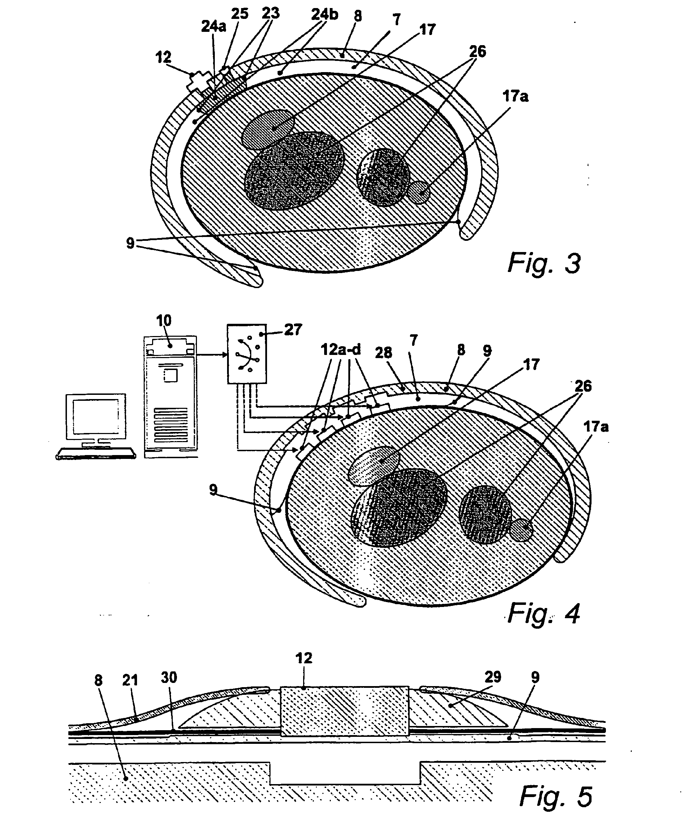

In Fig. 1 wird das neue Blutdruckmesssystem näher beschrieben. In Fig. 2 wird die neue Fingermanschette näher beschrieben. Fig. 3 zeigt eine Ausführung des Blutdruckmesssystems, wobei Druckaufnehmer als Arteriensignalaufnehmer verwendet werden. Fig. 4 zeigt eine Ausführung des Blutdruckmesssystems, wobei mehrere Signalaufnehmer als Arteriensignalaufnehmer verwendet werden. Fig. 5, Fig. 6 und Fig. 7 zeigen Detaildarstellungen der Arteriensignalaufnehmer.

In Fig. 1 ist mit 1 die Gasquelle gekennzeichnet, bei der es sich um eine Luftpumpe oder auch um eine Gaspatrone handeln könnte. Mit 2 ist ein Dämpfungsglied, z.B. ein Gasfilter eingezeichnet, das hochfrequente Unregelmäßigkeiten der Gaszufuhr, z.B. bei Verwendung einer Membranpumpe als Gasquelle ausgleicht und gleichzeitig als Staubfilter dient. Mit 3 ist die Druckkammer gekennzeichnet, wobei die Verbindung zur Gasquelle 1 durch ein Einlassventil 4 bewerkstelligt wird. Mit 5 ist das Auslassventil gekennzeichnet. Bei den Ventilen könnte es sich um konventionelle Proportionalventile handeln, besonders vorteilhaft ist jedoch die Verwendung von Ventilen mit sehr kurzen Schaltzeiten, wie sie z.B. durch piezoelektrische Elemente gegeben sind. Bei Schaltzeiten dieser piezoelektrischen Ventile von rund einer Millisekunde können hier Druckänderungen erreicht werden, die in einem Frequenzbereich bis 50 Hz liegen können. Bei der Verwendung von piezoelektrischen Ventilen lässt sich besonders einfach eine digitale Ansteuerung der Ventile durch einen Computer 10 erreichen, so dass den Ventilen über die digitale Ansteuerung Charakteristika gegeben werden können, die bei herkömmlichen Proportionalventilen bzw. bei der Zwangskoppelung von Auslass- und Einlassventil (wie z.B. in der US 4,406,289 A verwirklicht) nicht oder nur schwierig zu erreichen ist. So kann in der Druckkammer 3 jeder gewünschte Druckverlauf mit einer oberen Grenzfrequenz von ~50 Hz eingestellt und zusätzlich der Gasverbrauch niedrig gehalten werden.

Die Druckkammer 3 kann über die Leitungen 3a und 3b und ein Umschaltventil 6 mit zwei oder mehreren Druckpolstern 7, 7a verbunden sein, die der Arterienkompression dienen. Wenn nur ein Druckpolster 7 verwendet wird, kann das Umschaltventil 6 entfallen. Mit 8 ist die relativ starre Außenwand gekennzeichnet. Diese dient dazu, die Compliance des Druckpolsters 7 niedrig zu halten. Mit 9 ist eine verformbare Membran gekennzeichnet, die der Arterienkompression dient. Im speziellen Fall sind die Druckpolster 7, 7a im Querschnitt ringförmig ausgebildet, da sie für die Verwendung an den Fingern gedacht sind. Mit 11 ist eine starrer Positionierungsteil gekennzeichnet, mit dessen Hilfe die beiden Druckpolster 7, 7a verbunden sein können. Dies hat den Vorteil, dass die Lage der Druckpolster 7, 7a an den Fingern in relativ konstanter Ausrichtung gewährleistet ist. Dadurch ist auch eine konstante Lage der an der Begrenzung der Druckpolster 7, 7a angebrachten Arteriensignalaufnehmer 12 relativ zu der unter der verformbaren Membran 9 liegenden Arterie gewährleistet. Bei den Arteriensignalaufnehmern 12 kann es sich z.B. um Lichtquellen und Lichtaufnehmer (Arteriensignalempfänger 12a und Arteriensignalsender 12b) handeln, die den Fluss der Arterie messen, oder auch z.B. um Ultraschallaufnehmer oder Laser bzw. auch um Druckaufnehmer. So kann, gesteuert durch die Arteriensignalaufnehmer 12, die ebenfalls in Verbindung zum Computer 10 stehen, jederzeit im Druckpolster 7 bzw. 7a der gewünschte Druck hergestellt werden. Statt der hier gezeigten Druckpolster 7, 7a, die hier ringförmig gezeichnet sind, könnte natürlich auch jede andere Form, dem verwendeten Körperteil angepasst, verwendet werden. Sollte das Blutdruckmessgerät z.B. am Schädel über der Arteria Temporalis, verwendet werden, würde sich ein flacher Druckpolster 7 eignen.

Weiters ist irgendwo im Bereich des kommunizierenden Hohlraumes, gebildet aus Druckkammer 3 und Druckpolster 7, ein Druckaufnehmer 13 angebracht, der den Druck in der Druckkammer misst und zum Computer 10 weiterleitet. Bekanntlich entspricht ja der in der Druckkammer gemessene Druck bei geeigneter Steuerung durch die Arteriensignalaufnehmer 12 dem Arteriendruck. Vorteilsweise kann es sich bei dem gezeigten Druckaufnehmer 13 um einen Differenzdruckaufnehmer handeln. Das hat den Vorteil, dass die Druckmessung jederzeit auf die Höhendifferenz von Arterie, relativ zum Herzen korrigiert werden kann. Dazu ist eine flüssigkeitsgefüllte Leitung vorhanden, die bis in die Herzhöhe (schematisch in Fig. 1 mit einem Herzen symbolisiert) reicht. Vorteilhafterweise ist die flüssigkeitsgefüllte Leitung 14 mit einer Flüssigkeit gefüllt, die der Dichte von Blut entspricht. Die Flüssigkeit, mit der die Leitung 14 gefüllt ist, sollte einen geringen Ausgasungsgrad aufweisen (z.B. ölige Flüssigkeiten). Der Schlauch kann z.B. mittels einer Befestigungsvorrichtung 14a (z.B. Klettverschlussband, Druckknopf, Klemme, usw.) an der Extremität (z.B. Oberarm oder Kleidungsstück) in Herzhöhe befestigt werden. Am herznahen Ende der Leitung 14 kann eine frei flottierende Membran 14b angebracht sein, die die Flüssigkeit nicht ausfließen lässt, aber eine Bewegung der Flüssigkeitssäule zulässt. Über der frei flottierenden Membran 14b kann eine weitere luftdurchlässige, aber strapazfähige Membran 14c oder ein feinmaschiges Gitter 14c angebracht werden, die eine Verletzung der frei flottierenden Membran 14b verhindert.

Weiters kann ein weiterer Druckpolster 15 vorhanden sein, der über einer anderen Arterie, am besten über einer großen Arterie, zu liegen kommt, der mit einer weiteren Gasquelle 16 verbunden sein kann, um dort auf konventionelle Weise, z.B. oszillometrisch oder auskultatorisch den Blutdruck zu messen. Genauso könnte natürlich bei ausreichender Kapazität die Gasquelle 1 verwendet werden, was allerdings weitere Ventile (nicht gezeigt) notwendig machen würde. Bekanntlich arbeiten die konventionellen Blutdruckmessungen, wie auskultatorische oder oszillometrische Messung intermittierend, d.h. überlicherweise im Abstand von minimal einer halben bis einer Minute. Der weitere Druckpolster ist ebenfalls mit dem Computer 10 verbunden, so dass automatisch die Berechnung und Anzeige des fortlaufenden Arteriendruckes, wie er in der kleinen Arterie durch den Druckpolster 7 ermittelt wird, auf den wahren Wert des Blutdruckes in der gro-βen Arterie, wie er durch den Druckpolster 15 gemessen wird, korrigiert wird.

Die zweite Druckmessung über einer großen Arterie durch den Druckpolster 15 hat noch einen weiteren Vorteil: Bekanntlich muss für die kontinuierliche Druckmessung durch den Druckpolster 7 ständig der Druck im Druckpolster 7 dem mittleren Arteriendruck nachgeführt werden, d.h. der Arbeitspunkt muss nachgeregelt werden. Zur Nachregelung des Arbeitspunktes muss meistens die kontinuierliche Blutdruckmessung durch den Druckpolster 7 kurz unterbrochen werden. Durch die Druckmessung in einer anderen Arterie durch den Druckpolster 15 können nun diskontinuierlich große Änderungen des mittleren arteriellen Druckes entdeckt werden, und so automatisch und ohne Unterbrechung der kontinuierlichen Druckmessung durch den Druckpolster 7 der Arbeitspunkt laufen angepasst werden. So ist mit dem beschriebenen Blutdruckmessgerät erstmals eine kontinuierliche, ununterbrochene Aufzeichnung der wahren intraarteriellen Druckkurve möglich. Durch den automatischen Wechsel von einem Druckpolster 7 auf den anderen Druckpolster 7a durch das Umschaltventil 6 ist eine weitere Maßnahme gesetzt, mit der die Druckmessung nicht unterbrochen wird, da eine allfällige Schmerzbelastung des Patienten durch die laufende Messung am selben Ort verhindert wird.

Fig. 2 zeigt eine vorteilhafte Ausführung des Druckpolsters 7, der aus einer relativ starren Außenwand 8 besteht, die einerseits dem Druckpolster 7 die günstige geringe Compliance gibt, andererseits die starre Verbindung 11 zu dem benachbarten Druckpolster 7a erlaubt, der ähnlich aufgebaut ist. Innerhalb der relativ starren Außenwand 8 befindet sich die verformbare Membran 9, der im gezeigten Fall die Arteriensignalaufnehmer 12 aufliegen. So besteht keine störende Membran zwischen den Arteriensignalaufnehmer (Flussaufnehmern) 12 und den Arterien 17, die die Messung des Blutflusses beeinträchtigen könnte. Wie gesagt kann es sich bei den Flussaufnehmern um LED's kombiniert mit Lichtdetektoren, (z.B. Photodioden), Laser (bzw. Laserdioden) und Photodioden oder Ultraschallgebern und -empfängern handeln (Arteriensignalempfänger 12a und Arteriensignalsendern 12b). Ebenso ist die Verwendung von weiteren Drucksensoren (siehe Fig. 3) möglich. Für die Arteriensignalaufnehmer 12 sind vorteilhafterweise in der relativ starren Außenwand Vertiefungen 18 angebracht, in die die Arteriensignalaufnehmer 12 verschwinden können, wenn die verformbare Membran 9 an der starren Außenwand 8 eng anliegt. Dieses enge Anliegen ist deswegen sinnvoll, um die Compliance des Druckpolsters 7 möglich gering zu halten. Im gezeigten Fall sind die zwei Arteriensignalaufnehmer 12a und 12b zueinander im Winkel von 120 Grad angebracht, um ein optimales Signal zu gewährleisten, die Arterien 17 liegen zwar visavis des Fingerknochens 19 im Finger 20, was einem Winkel von 180 Grad im gezeigten Druckpolster 7 entspricht, das beste Signal ergibt sich, wie gesagt, bei einer Lage der Arteriensignalempfänger 12a und Arteriensignalsendern 12b von ca. 120 Grad zueinander, da gleichzeitig ein noch besserer und homogenerer Druck auf die Arterie ausgeübt werden kann. Dies ist deswegen der Fall, weil auf die Arterie 17 dann nur die verformbare Membran 9, nicht aber die Arteriensignalaufnehmer 12a und 12b zu liegen kommen, die nicht verformbar sind.

Im gezeigten Fall besteht die verformbare Membran 9 aus gasdichtem und flüssigkeitsdichtem Kunststoff. Um die Messung für den Patienten angenehmer zu machen, ist zusätzlich zwischen verformbarer Membran 9 und dem Körper ein hautfreundliches Gewebe 21 angebracht, das z.B. aus Nylon oder anderen Kunststoffgeweben, Baumwolle oder ähnlichem bestehen könnte. Das hautfreundliche Gewebe lässt dabei die Arteriensignalaufnehmer 12a und 12b frei, so dass das Signal nicht beeinträchtigt wird. Besonders vorteilhaft sind Stoffe, die sich leicht reinigen bzw. desinfizieren lassen. Weiters ist eine elektrische Abschirmung 22 vorgesehen, die elektrische Störungen von den Arteriensignalaufnehmern 12 fernhält. Im gezeigten Fall ist die elektrische Abschirmung 22 außen an der starren Außenwand 8 angebracht, sie könnte auch innerhalb der starren Außenwand 8 zu liegen kommen.

Um eine richtige Positionierung der Arteriensignalaufnehmer 12 über der Arterie 17 auch dann zu gewährleisten, wenn nur ein Druckpolster 7 vorhanden ist, kann es günstig sein, den starren Positionierungsteil 11 an der starren Außenwand 8 auch dann anzubringen, wenn nur ein Druckpolster verwendet wird. Der starre Positionierungsteil 11 ist dann den benachbarten Körperstrukturen (beim Finger z.B. die Nachbarfinger, Handrücken, Handfläche; im Falle des Daumens dem Tenar, nicht dargestellt) nachgeformt und könnte damit auch weiter die Ringform behalten bzw. Teile eines Ringes bilden.

Wie in Fig. 3 gezeigt, kann es günstig sein, Druckaufnehmer als Arteriensignalaufnehmer 12 in der starren Außenwand 8 zu lagern. In diesem gezeigten Fall kann es günstig sein, das kommunizierende System, welches aus der Druckkammer 3 und Druckpolster 7 besteht, durch zusätzliche leicht verformbare Septen 23 zu teilen, die getrennte Bereiche 24a und 24b in der Druckkammer schaffen. Der im Bereich des Arteriensignalaufnehmers befindliche Bereich 24a kann dann mit einem andern Medium, nämlich mit Flüssigkeit gefüllt sein, um die Übertragung der von der Arterie ausgestrahlten Signale besser zum Arteriensignalaufnehmer überzuleiten. Mit 25 ist dabei eine Füll- bzw. Entlüftungsöffnung gezeigt, die verschlossen werden kann und die sich in der relativ starren Außenwand 8 befindet, über die der Sektor 24a mit einer Flüssigkeit gefüllt werden kann. Diese Ausführungsform hat den Vorteil, dass der Arteriensignalaufnehmer 12 im konkreten Fall z.B. auch ein hochauflösender Druckaufnehmer sein kann, der die unverfälschten, nicht gedämpften Signale von der am Knochen 26 anliegenden Arterie 17 aufnehmen kann, ohne diese mechanisch zu beeinträchtigen. So kann die fortlaufende Pulskurve mit hoher Auflösung fortlaufend aufgezeichnet werden, während über die flexiblen Septen 23 ein genau bekannter Druck der Arterie 17 aufgebracht werden kann. So kann die Arterienwand entspannt werden, und eine unverfälschte Pulskurve kontinuierlich registriert werden.

Bei der hier verwendeten Ausführungsform kann einerseits mit dem Druckaufnehmer 13, der mit dem Sektor 24b des Druckpolsters 7 in Verbindung steht, auch in bekannter Weise oszillometrisch der Blutdruck gemessen werden, und dann bei Kenntnis des systolischen, diastolischen und mittleren arteriellen Druckes, jeder gewünschte Druck in Relation zum systolischen, diastolischen und mittleren arteriellen Druck im Druckpolster 7 und damit auch im flüssigkeitsgefüllten Bereich 24a hergestellt werden, um so bei genau definierten Druckverhältnissen die Pulskurve aufzuzeichnen und damit eine unblutige fortlaufende Blutdruckaufzeichnung zu ermöglichen. Genauso können natürlich auch andere Arteriensignalaufnehmer 12 (Empfänger 12a und Sender 12b), wie z.B. lichtempfindliche Aufnehmer und LED's in der starren Außenwand angebracht sein.

Wie in Fig. 4 gezeigt, können auch mehrere Arteriensignalaufnehmer (12a-d) vorhanden sein, wobei eine Multiplexschaltung 27 und der verwendete Rechner 10 die Auswahl des optimal platzierten Arteriensignalaufnehmers 12a-d vornimmt, um ein optimales Arteriensignal zu erhalten. Dies ist besonders dann günstig, um bei einer veränderlich Position der Arterie von Individuum zu Individuum trotzdem eine störungsfreie Signalaufzeichnung zu ermöglichen. Ideal wäre es, statt der Lokalisation des Druckpolsters 7 über einer kleinen Arterie, z.B. der Fingerarterie, mit der Notwendigkeit der Nacheichung der Messung über einen weiteren Druckpolster 15, der über einer großen Arterie liegt, von vornherein nur einen Druckpolster 7 über einer großen Arterie zu verwenden, die eine kontinuierliche Druckmessung erlaubt und dabei auch die Absolutwerte richtig ermittelt werden. So eine Arterie ist z.B. die Arterias radialis oder temporalis, die einerseits groß genug ist, um noch repräsentativ für die großen Arterien zu sein, andererseits aber noch eine Arteriensignalaufzeichnung, z.B. auch eine Flussmessung durch eine Durchstrahlung oder Reflexion an dem darunter liegenden Knochen 26 (z.B. Radius oder Schädelknochen) durch den Arteriensignalsender 12b ausgestrahlten Wellen erlaubt. Die Arteria radialis hat z.B. auch den zusätzlichen Vorteil, dass noch eine weiter Arterie, nämlich die weitere Arterie 17a im Beispielsfall die Arteria ulnaris vorhanden ist. Für die Messung muss nur die Arterie 17 durch den Druckpolster 7 komprimiert werden, nicht jedoch die weitere Arterie 17a und daher ist der Blutfluss in die Extremität nicht unterbrochen. Dazu muss nur die verformbare Membran 9 aufblasbar mit einem Sektor 28 der starren Außenwand 8 in dem Bereich verbunden sein, der über der untersuchten Arterie 17 liegt, während die weitere Arterie 17a durch die verformbare Membran 9 nicht komprimiert wird.

Fig. 5 zeigt eine praktische Ausführung der Vorrichtung, wie sie dann von Vorteil ist, wenn die Arteriensignalaufnehmer (Empfänger 12a und Sender 12b) der verformbaren Membran 9 aufgelagert sind. In diesem Fall ist die verformbare Membran, die z.B. aus Latex bestehen könnte, nicht unterbrochen, sondern der Arteriensignalaufnehmer 12 ist in eine verformbare Linse 29, bevorzugt aus dem gleichen Material wie die verformbare Membran 9 eingegossen, die an der Membran 9 befestigt (z.B. geklebt oder vulkanisiert) ist. Die elektrischen Zuleitungen 30 sind dabei zwischen der verformbaren Membran 9 und dem hautfreundlichen Gewebe 21 geführt, so dass diese Zuleitungen auch mechanisch geschützt und isoliert zum Rechner 10 geführt werden können.

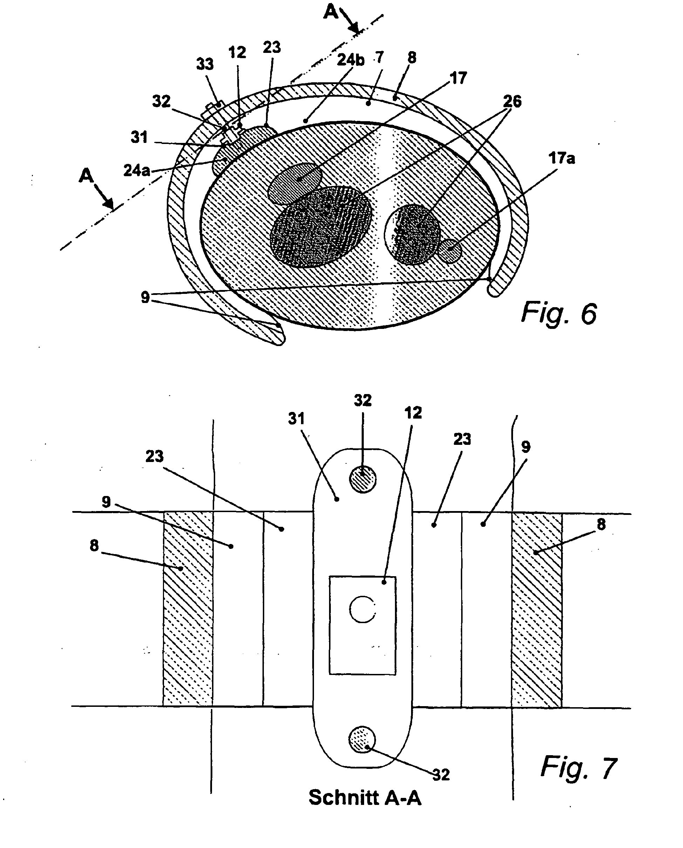

Fig. 6 zeigt eine weitere Ausführungsform der geplanten Vorrichtung, bei der die Arteriensignalaufnehmer 12 auf einer Leiste 31 angebracht sind, wobei die Leiste 31 einen Teil des Septums 23 darstellt, der den gasgefüllten Bereich 24b vom flüssigkeitsgefüllten Bereich 24a trennt. Auf der vom Körper abgewandten Seite ist dabei der gasgefüllte Bereich 24b durchgezogen, so dass bei Erhöhung des Druckes in der Druckkammer 3 (und somit im gasgefüllten Bereich 24b) die Arteriensignalaufnehmer ihre Position zur Arterie 17 nicht oder nur wenig verändern und jedenfalls nicht vom Körper abgehoben werden. Dadurch wird unabhängig vom Druck in der Druckkammer 3 immer ein optimales Signal der Arteriensignalaufnehmer 12 erreicht. Damit die Arteriensignalaufnehmer 12 in der Leiste 31 nicht kippen können, kann zusätzlich ein, besser zwei Steher 32 fest mit der Leiste 31 verbunden sein, die in der relativ starren Außenwand 8 beweglich, z.B. in Führungsöffnungen 33 gelagert sind. Damit eine optimale Druckübertragung ohne Druckverlust vom Bereich 24b zum Bereich 24a möglich ist, ist die Leiste 31 schmal, so dass das Septum 23 den Druck vom Bereich 24b von mehreren Seiten auf den Bereich 24a übertragen kann.

Wie in Fig. 7 gezeigt, sind die Steher 32 dabei außerhalb des gasgefüllten Bereiches 24b des Druckpolsters 7 vorbeigeführt, damit der Druckpolster 7 nicht unterbrochen werden muss.

## Patentansprüche

1. Blutdruckmessgerät für die kontinuierliche plethysmographische Blutdruckmessung mit zumindest einem aufblasbaren Druckpolster (7), der an einem eine Arterie enthaltenden Körperteil befestigbar ist, mit dem Körperteil zugeordneten Arteriensignalaufnehmern (12; 12a, 12b) zum Bestimmen des Durchflusses, mit einer ventilgesteuerten Druckkammer (3), welche mit einer Gasquelle (1) und mit dem aufblasbaren Druckpolster (7) in Verbindung steht, mit einem Druckaufnehmer (13) zur Messung des Druckes in der Druckkammer (3) bzw. dem Druckpolster (7) und mit einem Rechner (10), wobei die Ventilsteuerung der Druckkammer (3) in Abhängigkeit des Signals der Arteriensignalaufnehmer (12; 12a, 12b) erfolgt, **dadurch gekennzeichnet, dass** die Druckkammer (3) mit je einem separaten Einlass- (4) und Auslassventil (5) ausgestattet ist.

2. Blutdruckmessgerät nach Anspruch 1, **dadurch gekennzeichnet, dass** die Arteriensignalaufnehmer (12; 12a, 12b) in eine verformbare Membran (9) des Druckpolsters (7) eingearbeitet sind und dass eine starre Außenwand (8) des Druckpolsters (7) vorhanden ist, in der Vertiefungen (18) für die Arteriensignalaufnehmer (12; 12a, 12b) vorhanden sind.

3. Blutdruckmessgerät nach Anspruch 1, **dadurch gekennzeichnet, dass** die Arteriensignalaufnehmer (12) in die dem Körper teil abgewandte Seite des Druckpolsters (7) eingearbeitet sind und dass der Druckpolster (7) durch zumindest ein verformbares Septum (23) in getrennte Bereiche (24a, 24b) unterteilt ist, welche Bereiche mit unterschiedlichen Medien, z. B. mit Gas oder Flüssigkeit, gefüllt sind.

4. Blutdruckmessgerät nach Anspruch 3, **dadurch gekennzeichnet, dass** die Arteriensignalaufnehmer (12) in die dem Körperteil abgewendete Seite des Septums (23) aufgenommen sind.

5. Blutdruckmessgerät nach Anspruch 4, **dadurch gekennzeichnet, dass** die Arteriensignalaufnehmer (12) auf einer Leiste (31) angeordnet sind.

6. Blutdruckmessgerät nach Anspruch 5, **dadurch gekennzeichnet, dass** die Leiste (31) beweglich gegenüber der starren Außenwand (8) des Druckpolsters (7) gelagert ist.

7. Blutdruckmessgerät nach Anspruch 6, **dadurch gekennzeichnet, dass** die Leiste (31) Steher (32) aufweist, die in Führungsöffnungen (33) in der starren Außenwand (8) beweglich geführt sind.

8. Blutdruckmessgerät nach einem der Ansprüche 1 bis 7, **dadurch gekennzeichnet, dass** der Druckpolster (7) ringförmig ist.

9. Blutdruckmessgerät nach einem der Ansprüche 2 bis 8, **dadurch gekennzeichnet, dass** die starre Außenwand (8) des Druckpolsters (7) zumindest einen starren Positionierungsteil (11) aufweist.

10. Blutdruckmessgerät nach Anspruch 9, **dadurch gekennzeichnet, dass** der starre Positionierungsteil (11) einen Ring bzw. Teile eines Ringes aufweist.

11. Blutdruckmessgerät nach Anspruch 10, **dadurch gekennzeichnet, dass** sich im Ring des Positionierungsteiles (11) ein weiterer Druckpolster (7a) befindet, und dass der Druckkammer (3) ein Umschaltventil (6) vorgeschaltet ist, womit abwechselnd einer der Druckpolster (7, 7a) mit Druck beaufschlagbar ist.

12. Blutdruckmessgerät nach einem der Ansprüche 1 bis 11, **dadurch gekennzeichnet, dass** zur Befestigung an einem weiteren Körperteil mit einer Arterie ein weiterer Druckpolster (15) für eine konventionelle intermittierende oszillatorische oder auskultatorische Blutdruckmessung vorgesehen ist, wobei der Arbeitspunkt für die kontinuierliche Blutdruckmessung durch den weiteren Druckpolster (15) regelbar ist.

13. Blutdruckmessgerät nach Anspruch 12, **dadurch gekennzeichnet, dass** der Rechner (10) laufend den kontinuierlich gemessenen Blutdruck auf den durch den weiteren Druckpolster (15) gemessenen intermittierenden Blutdruck normiert.

14. Blutdruckmessgerät nach einem der Ansprüche 1 bis 13, **dadurch gekennzeichnet, dass** mehrere Arteriensignalaufnehmer (12) vorhanden sind, aus denen der Rechner (10) den oder die Arteriensignalaufnehmer (12) mit der besten Signalcharakteristik heraussucht und ansteuert.

15. Blutdruckmessgerät nach einem der Ansprüche 2 bis 14, **dadurch gekennzeichnet, dass** nur ein Sektor (28) der starren Außenwand (8) des Druckpolsters (7), der die Arteriensignalaufnehmer (12) beinhaltet, mit einer verformbaren Membran (9) ausgekleidet ist.

16. Blutdruckmessgerät nach einem der Ansprüche 1, 2 oder 8 bis 15, **dadurch gekennzeichnet, dass** die Arteriensignalaufnehmer (12a, 12b) im Winkel von ca. 120° zueinander angeordnet sind.

17. Blutdruckmessgerät nach einem der Ansprüche 1 bis 16, **dadurch gekennzeichnet, dass** der Druckaufnehmer (13) ein Differentialdruckaufnehmer (13) ist, dessen einer Teil mit der Druckkammer (3) bzw. mit dem Druckpolster (7) und dessen anderer Teil mit einer flüssigkeitsgefüllten Leitung (14) in Druckverbindung steht.

18. Blutdruckmessgerät nach Anspruch 17, **dadurch gekennzeichnet, dass** die flüssigkeitsgefüllte Leitung (14) mit einer Flüssigkeit mit niedrigem Dampfdruck gefüllt ist.

19. Blutdruckmessgerät nach Anspruch 17 oder 18, **dadurch gekennzeichnet, dass** die flüssigkeitsgefüllte Leitung (14) an ihrer dem Differentialdruckaufnehmer (13) abgewendeten Seite mit einer leicht verformbar flottierenden Membran (14b) verschlossen ist.

20. Blutdruckmessgerät nach Anspruch 19, **dadurch gekennzeichnet, dass** die leicht verformbare Membran (14b) an ihrer Außenseite mit einer strapazfähigen luftdurchlässigen Membran (14c) umgeben ist.

21. Blutdruckmessgerät nach einem der Ansprüche 17 bis 20, **dadurch gekennzeichnet, dass** die flüssigkeitsgefüllte Leitung (14) an ihrem herznahen Ende mit einer Befestigungsvorrichtung (14a) ausgestattet ist.

22. Blutdruckmessgerät nach einem der Ansprüche 1 bis 21, **dadurch gekennzeichnet, dass** als Gasquelle (1) eine Gaspatrone eingesetzt ist.

23. Blutdruckmessgerät nach einem der Ansprüche 1, 2 oder 8 bis 22, **dadurch gekennzeichnet, dass** die Arteriensignalaufnehmer (12) in eine verformbare Linse (29) eingelagert sind und dass elektrische Zuleitungen (30) zu den Arteriensignalaufnehmern (12) außerhalb des Druckpolsters (7) und innerhalb eines hautfreundlichen Gewebes (21) geführt sind.

## Claims

1. A sphygmomanometer for continuous plethysmographic measurement of blood pressure, comprising at least one inflatable pressure pad (7) which can be attached to a body part containing an artery, with arterial signal sensors (12; 12a, 12b) associated with the body part for determining the throughflow, with a valve-controlled pressure chamber (3) connected to a gas source (1) and to the inflatable pressure pad (7), with a pressure sensor (13) for measuring the pressure in the pressure chamber (3) or the pressure pad (7), and with a computer (10), with valve control of the pressure chamber (3) being realized depending on the signal of the arterial signal sensors (12; 12a, 12b), **characterized in that** the pressure chamber (3) is fitted with a separate inlet valve (4) and outlet valve (5) each.

2. A sphygmomanometer according to claim 1, **characterized in that** the arterial signal sensors (12; 12a, 12b) are incorporated in the deformable membrane (9) of pressure pad (7) and that a rigid outer wall (8) of the pressure pad is available, containing depressions (18) for the arterial signal sensors (12; 12a, 12b).

3. A sphygmomanometer according to claim 1, **characterized in that** the arterial signal sensors (12) are incorporated in the side of the pressure pad (7) averted from the body part and that the pressure pad (7) is separated by at least one deformable septum (23) into separate areas (24a, 24b), which areas are filled with different media, for example gas or fluid.

4. A sphygmomanometer according to claim 3, **characterized in that** the arterial signal sensors (12) are received in the side of the septum (23) averted from the body part.

5. A sphygmomanometer according to claim 4, **characterized in that** the arterial signal sensors (12) are arranged on a strip (31).

6. A sphygmomanometer according to claim 5, **characterized in that** the strip (31) is mounted movably opposite the rigid outer wall (8) of the pressure pad (7).

7. A sphygmomanometer according to claim 6, **characterized in that** the strip (31) has spacer elements (32) which are guided movably in guide openings (33) in the rigid outer wall (8).

8. A sphygmomanometer according to one of claims 1 to 7, **characterized in that** the pressure pad (7) is annular in shape.

9. A sphygmomanometer according to one of claims 2 to 8, **characterized in that** the rigid outer wall (8) of the pressure pad (7) comprises at least one rigid positioning component (11).

10. A sphygmomanometer according to claim 9, **characterized in that** the rigid positioning component (11) comprises a ring or parts of a ring.

11. A sphygmomanometer according to claim 10, **characterized in that** an additional pressure pad (7a) is located in the ring of the positioning component (11), and that a reversing valve (6) is arranged upstream of the pressure chamber (3) with which one of the pressure pads (7, 7a) can be pressurized alternately.

12. A sphygmomanometer according to one of claims 1 to 11, **characterized in that** a further pressure pad (15) is provided for fastening to another body part with an artery for conventional, intermittent, oscillatory or auscultatoric measuring of blood pressure, with the operating point for continuous measuring of blood pressure being regulatable by the further pressure pad (15).

13. A sphygmomanometer according to claim 12, **characterized in that** the computer (10) continuously standardizes the continually measured blood pressure to the blood pressure measured intermittently by the further pressure pad (15).

14. A sphygmomanometer according to one of claims 1 to 13, **characterized in that** several arterial signal sensors (12) are provided, from which the computer (10) searches out and controls the arterial signal sensor(s) (12) with the best signal characteristic.

15. A sphygmomanometer according to one of claims 2 to 14, **characterized in that** only one sector (28) of the rigid outer wall (8) of the pressure pad (7), containing the arterial signal sensors (12), is lined with a deformable membrane (9).

16. A sphygmomanometer according to one of claims 1, 2 or 8 to 15, **characterized in that** the arterial signal sensors (12a, 12b) are applied at an angle of ca. 120° relative to one another.

17. A sphygmomanometer according to one of claims 1 to 16, **characterized in that** the pressure sensor (13) is a differential pressure sensor (13), whose one part is in pressure connection with the pressure chamber (3) or to the pressure pad (7) and whose other part is connected to a fluid-filled line (14).

18. A sphygmomanometer according to claim 17, **characterized in that** the fluid-filled line (14) is filled with a fluid with a low vapor pressure.

19. A sphygmomanometer according to claim 17 or 18, **characterized in that** the fluid-filled line (14) is sealed at its side averted from the differential pressure sensor (13) with an easily deformable, freely floating membrane (14b).

20. A sphygmomanometer according to claim 19, **characterized in that** the easily deformable membrane (14b) is surrounded on its outside by a durable, but air-permeable membrane (14c).

21. A sphygmomanometer according to one of claims 17 to 20, **characterized in that** the fluid-filled line (14) is fitted with a fastening device (14a) at its end close to the heart.

22. A sphygmomanometer according to one of claims 1 to 21, **characterized in that** a gas cartridge is used as a gas source (1).

23. A sphygmomanometer according to one of claims 1, 2 or 8 to 22, **characterized in that** the arterial signal sensors (12) are mounted in a deformable lens (29) and that electrical wires (30) are guided to the arterial signal sensors (12) outside the pressure pad (7) and inside the skin-compatible tissue (21).

## Revendications

1. Appareil de mesure de la pression artérielle permettant de mesurer la pression artérielle en continu par pléthysmographie, comportant au moins un coussin de pression (7) gonflable pouvant être fixé sur une partie du corps proche d'une artère, des capteurs de signal artériel (12 ; 12a ; 12b) associés à la partie du corps pour déterminer l'écoulement, une chambre de compression (3) commandée par vanne qui communique avec une source de gaz (1) et avec le coussin de pression (7) gonflable, un capteur de pression (13) pour mesurer la pression régnant dans la chambre de compression (3) ou dans le coussin de pression (7), et un ordinateur (10), la commande par vanne de la chambre de compression (3) se produisant en fonction du signal des capteurs de signal artériel (12 ; 12a ; 12b),
**caractérisé en ce que**
la chambre de compression (3) est équipée d'une vanne d'admission (4) séparée et d'une vanne de décharge (5).

2. Appareil de mesure de la pression artérielle selon la revendication 1,
**caractérisé en ce que**
les capteurs de signal artériel (12 ; 12a ; 12b) sont montés dans une membrane déformable (9) du coussin de pression (7), et une paroi extérieure rigide (8) du coussin de pression (7) comporte des creux (18) destinés aux capteurs de signal artériel (12 ; 12a ; 12b).

3. Appareil de mesure de la pression artérielle selon la revendication 1,
**caractérisé en ce que**
les capteurs de signal artériel (12) sont montés dans la face du coussin de pression (7) détournée de la partie du corps et **en ce que** le coussin de pression (7) est divisé en diverses zones (24a, 24b) au moyen d'au moins un septum déformable (23), les zones étant remplies de milieux différents, par ex. de gaz ou de liquide.

4. Appareil de mesure de la pression artérielle selon la revendication 3,
**caractérisé en ce que**
les capteurs de signal artériel (12) se logent dans la face du septum (23) détournée de la partie du corps.

5. Appareil de mesure de la pression artérielle selon la revendication 4,
**caractérisé en ce que**
les capteurs de signal artériel (12) sont disposés sur une réglette (31).

6. Appareil de mesure de la pression artérielle selon la revendication 5,
**caractérisé en ce que**
la réglette (31) est montée de manière mobile par rapport à la paroi extérieure rigide (8) du coussin de pression (7).

7. Appareil de mesure de la pression artérielle selon la revendication 6,
**caractérisé en ce que**
la réglette (31) présente des montants (32) qui passent de manière mobile par des ouvertures de guidage (33) pratiquées dans la paroi extérieure rigide (8).

8. Appareil de mesure de la pression artérielle selon l'une des revendications 1 à 7,
**caractérisé en ce que**
le coussin de pression (7) est annulaire.

9. Appareil de mesure de la pression artérielle selon l'une des revendications 2 à 8,
**caractérisé en ce que**
la paroi extérieure rigide (8) du coussin de pression (7) présente au moins une partie de positionnement rigide (11).

10. Appareil de mesure de la pression artérielle selon la revendication 9,
**caractérisé en ce que**
la partie de positionnement rigide (11) présente un anneau ou des parties d'un anneau.

11. Appareil de mesure de la pression artérielle selon la revendication 10,
**caractérisé en ce qu'**
un coussin de pression supplémentaire (7a) se trouve dans l'anneau de la partie de positionnement (11), et une vanne d'inversion (6) située en amont de la chambre de compression (3), permet alternativement de soumettre à la pression l'un des coussins de pression (7, 7a).

12. Appareil de mesure de la pression artérielle selon l'une des revendications 1 à 11,
**caractérisé par**
un coussin de pression supplémentaire (15) à fixer sur une autre partie du corps proche d'une artère pour une mesure de la pression artérielle conventionnelle intermittente oscillatoire ou auscultatoire, le point de travail servant à la mesure de la pression artérielle continue étant réglable grâce au coussin de pression supplémentaire (15).

13. Appareil de mesure de la pression artérielle selon la revendication 12,
**caractérisé en ce que**
l'ordinateur (10) norme en permanence la pression artérielle mesurée en continu à la pression artérielle intermittente mesurée par le coussin de pression supplémentaire (15).

14. Appareil de mesure de la pression artérielle selon l'une des revendications 1 à 13,
**caractérisé par**
plusieurs capteurs de signal artériel (12), l'ordinateur (10) recherchant et commandant le ou les capteurs de signal artériel (12) comportant la meilleure caractéristique de signal.

15. Appareil de mesure de la pression artérielle selon l'une des revendications 2 à 14,
**caractérisé en ce que**
seul un secteur (28) de la paroi extérieure rigide (8) du coussin de pression (7), qui contient les capteurs de signal artériel (12), est revêtu d'une membrane déformable (9).

16. Appareil de mesure de la pression artérielle selon l'une des revendications 1, 2 ou 8 à 15,
**caractérisé en ce que**
les capteurs de signal artériel (12a, 12b), sont disposés l'un par rapport à l'autre dans un angle d'environ 120°.

17. Appareil de mesure de la pression artérielle selon l'une des revendications 1 à 16,
**caractérisé en ce que**
le capteur de pression (13) est un capteur de pression différentielle (13) dont une partie est en communication par pression avec la chambre de compression (3) ou avec le coussin de pression (7) et l'autre partie est en communication par pression avec une conduite remplie de liquide (14).

18. Appareil de mesure de la pression artérielle selon la revendication 17,
**caractérisé en ce que**
la conduite remplie de liquide (14) est remplie d'un liquide à faible pression de vapeur.

19. Appareil de mesure de la pression artérielle selon la revendication 17 ou 18,
**caractérisé en ce que**
la conduite remplie de liquide (14) est obturée sur son côté détourné du capteur de pression différentielle (13) par une membrane flottante (14b) facilement déformable.

20. Appareil de mesure de la pression artérielle selon la revendication 19,
**caractérisé en ce que**
la membrane (14b) facilement déformable est entourée sur son côté extérieur par une membrane (14c) résistante perméable à l'air.

21. Appareil de mesure de la pression artérielle selon l'une des revendications 17 à 20,
**caractérisé en ce que**
la conduite remplie de liquide (14) est munie d'un dispositif de renfort (14a) à son extrémité proche du coeur.

22. Appareil de mesure de la pression artérielle selon l'une des revendications 1 à 21,
**caractérisé en ce que**
la source de gaz (1) utilisée est une cartouche de gaz.

23. Appareil de mesure de la pression artérielle selon l'une des revendications 1, 2 ou 8 à 22,
**caractérisé en ce que**
les capteurs de signal artériel (12) sont incorporés dans une lentille (29) déformable, et des lignes d'alimentation électrique (30) sont acheminées aux capteurs de signal artériel (12) en dehors du coussin de pression (7) et à l'intérieur d'un tissu (21) compatible avec la peau.
